(19) Europäisches Patentamt
European Patent Office
Office européen des brevets

(11) Veröffentlichungsnummer: **0 212 480**
**A1**

# (12) EUROPÄISCHE PATENTANMELDUNG

(21) Anmeldenummer: **86110951.0**

(22) Anmeldetag: **08.08.86**

(51) Int. Cl.⁴: **C 07 C 49/788**, C 07 C 45/45

(30) Priorität: **23.08.85 DE 3530145**

(43) Veröffentlichungstag der Anmeldung: **04.03.87 Patentblatt 87/10**

(84) Benannte Vertragsstaaten: **DE FR GB IT**

(71) Anmelder: **RIEDEL-DE HAEN AKTIENGESELLSCHAFT, Wunstorfer Strasse 40, D-3016 Seelze 1 (DE)**

(72) Erfinder: **Bartels, Günter, Dr., Leimenweg 31, D-3300 Braunschweig (DE)**
Erfinder: **Schulz, Joachim, Königsberger Strasse 7, D-3255 Pohle (DE)**

(74) Vertreter: **Beck, Bernhard et al, HOECHST AKTIENGESELLSCHAFT Zentrale Patentabteilung Postfach 80 03 20, D-6230 Frankfurt/Main 80 (DE)**

(54) **Verfahren zur Herstellung eines 6-Acyl-2-alkylnaphthalins.**

(57) 6-Acyl-2-alkylnaphthaline der Formel

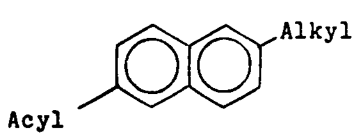

werden üblicherweise durch Acylieren von 2-Alkylnaphthalinen in Gegenwart von Lösungsmitteln mit Hilfe von Katalysatoren hergestellt. Diese Friedel-Crafts-Reaktion führt zu Gemischen von isomeren Ketonen. Besonders vorteilhaft ist die Durchführung der Umsetzung in überschüssigem wasserfreien Fluorwasserstoff ohne Einsatz von Katalysatoren. 6-Acyl-2-alkylnaphthaline eignen sich als Ausgangsmaterial zur Herstellung von Arzneimitteln und Kunststoffen.

EP 0 212 480 A1

RIEDEL-DE HAEN AG HOE 85 D 004

Verfahren zur Herstellung eines 6-Acyl-2-alkylnaphthalins

Die Erfindung bezieht sich auf ein Verfahren zur Herstellung eines 6-Acyl-2-alkylnaphthalins durch Acylierung eines 2-Alkylnaphthalins in Gegenwart eines Lösungsmittels und das nach diesem Verfahren erhaltene Produkt.

Es ist bekannt, daß 6-Acetyl-2-methylnaphthalin erhältlich ist durch Reaktion von Acetylchlorid mit 2-Methylnaphthalin in Gegenwart von Bortrifluorid als Katalysator und Tetrachlorethan als Lösungsmittel (US-Patentschrift 2 644 841). Das Produkt dient als Ausgangsmaterial zur Synthese von 2,6-Naphthalinsäure, die ihrerseits zur Herstellung von Polyesterharzen verwendbar ist.

Ferner ist bekannt, daß durch Friedel-Crafts-Acylierung von 2-Methylnaphthalin Ketongemische erhalten werden, die je nach den einzelnen Reaktionsbedingungen unterschiedliche Mengen an 6-Acetyl-2-methylnaphthalin enthalten (J. Chem. Soc. 1972 (14), 1781 - 1788). Die Anteile des 2,6-Isomers liegen zwischen 7,4 und 73 Prozent; Gesamtausbeuten von 74 und 65 Prozent mit hohem Anteil an 2,6-Isomer lassen sich nur bei Verwendung von Nitromethan und Nitrobenzol erzielen.

Die Synthese bestimmter 6-Acyl-2-alkylnaphthaline durch Acylierung von 2-Methylnaphthalin mit Acylfluoriden in Gegenwart von Aluminiumtrichlorid oder Bortrifluorid und Methylenchlorid als Lösungsmittel ist ebenfalls beschrieben (J. Org. Chem. 1984 (49), 384 - 385). Als Acylierungsmittel sind erwähnt Isobuttersäurefluorid, Dekancarbonsäurefluorid, Acetylfluorid und Propionylfluorid; diese Fluoride können angeblich auch in situ durch Reaktion von Acylchloriden mit wasserfreiem Fluorwasserstoff hergestellt werden, ohne daß hierzu jedoch Einzelheiten angegeben sind. Die Ausbeuten an 2,6-Isomer liegen bei 70 bis 83 Prozent.

Aufgabe der Erfindung ist die Entwicklung eines Verfahrens zur Herstellung von 6-Acyl-2-alkylnaphthalinen, das in wirtschaftlicher Weise ohne den Einsatz von Katalysatoren durchgeführt werden kann.

Die Erfindung betrifft ein Verfahren zur Herstellung eines 6-Acyl-2-alkylnaphthalins durch Acylierung eines 2-Alkylnaphthalins in Gegenwart eines Lösungsmittels und ist dadurch gekennzeichnet, daß ein 2-Alkylnaphthalin der Formel (I)

(I) 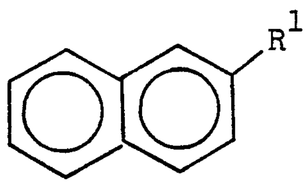 ,

in der $R^1$ einen niederen Alkylrest bedeutet, in Gegenwart von wasserfreiem Fluorwasserstoff mit einem Säurederivat der Formel (II)

(II) $R^2$-CO-X ,

in der $R^2$ einen niederen Alkylrest und X eine Abgangsgruppe bedeutet, umgesetzt wird, wobei das Molverhältnis von Säurederivat zu Fluorwasserstoff 1 : 5 bis 1 : 30 und das Molverhältnis von 2-Alkylnaphthalin zu Säurederivat 1 : 1 bis 1 : 2,5 beträgt.

Als Ausgangsmaterial für das erfindungsgemäße Verfahren wird ein 2-Alkylnaphthalin der Formel (I) verwendet,

(I) 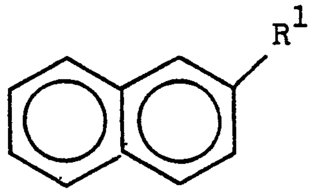

in der $R^1$ einen niederen Alkylrest, vorzugsweise einen Alkylrest mit 1 bis 4 Kohlenstoffatomen, bedeutet. Geeignete Verbindungen sind beispielsweise 2-Methylnaphthalin, 2-Ethylnaphthalin, 2-n-Propylnaphthalin, 2-iso-Propylnaphthalin und 2-tert.-Butylnaphthalin.

Die Acylierung des 2-Alkylnaphthalins wird mit einem Säurederivat der Formel (II) durchgeführt,

(II) $R^2$-CO-X

in der $R^2$ einen niederen Alkylrest, vorzugsweise einen Alkylrest mit 1 bis 4 Kohlenstoffatomen, bedeutet und X eine Abgangsgruppe darstellt. Die Abgangsgruppe, die als funktionelle Gruppe des Säurederivats mit einem Wasserstoffatom am C6 des Alkylnaphthalins reagiert, ist insbesondere ein Halogenatom, vorzugsweise ein Chlor- oder Bromatom, eine Hydroxylgruppe, eine Alkoxygruppe mit 1 bis 4 Kohlenstoffatomen oder eine Acyloxygruppe mit 2 bis 5 Kohlenstoffatomen. Geeignete Verbindungen sind beispielsweise Säurehalogenide, vorzugsweise Säurechloride und Säurebromide wie Acetylchlorid, Propionylchlorid, Butyrylchlorid, Isobutyrylchlorid, Acetylbromid, Propionylbromid, Butyrylbromid und Isobutyrylbromid, ferner Carbonsäuren wie Essigsäure, Propionsäure, Buttersäure und Isobuttersäure, Carbonsäureanhydride wie Essigsäureanhydrid, Propionsäureanhydrid, Buttersäureanhydrid und Isobuttersäureanhydrid, sowie Carbonsäureester von niederen Alkoholen wie Essigsäuremethylester, Essigsäureethylester, Essigsäurepropylester, Propionsäuremethylester, Propionsäureethylester, Propionsäurepropylester, Buttersäuremethylester, Buttersäureethylester und Buttersäurepropylester.

Das Molverhältnis von 2-Alkylnaphthalin zu Säurederivat beträgt im allgemeinen 1 : 1 bis 1 : 2,5, vorzugsweise 1 : 1,2 bis 1 : 2.

Die Umsetzung des 2-Alkylnaphthalins mit dem Säurederivat wird in überschüssigem wasserfreiem Fluorwasserstoff durchgeführt, der auch als Reaktionsmedium (Lösungsmittel) dient. Das Molverhältnis von Säurederivat zu Fluorwasserstoff liegt im allgemeinen im Bereich von 1 : 5 bis 1 : 30, vorzugsweise 1 : 10 bis 1 : 20. Die Reaktion wird bei einer Temperatur von 0 bis 80°C durchgeführt. Dabei ist es empfehlenswert, im Temperaturbereich von 0 bis 20°C unter Normaldruck und im Temperaturbereich von mehr als 20 bis 80°C unter erhöhtem Druck zu arbeiten, vorzugsweise bei einem Druck von 1,5 bis 5 bar.

Eine bevorzugte Ausführungsform des erfindungsgemäßen Verfahrens besteht darin, daß zunächst das Säurederivat bei einer Temperatur von 0 bis 5°C mit dem Fluorwasserstoff vermischt wird und anschließend das 2-Alkylnaphthalin zugegeben und die Umsetzung bei einer Temperatur von 0 bis 80°C durchgeführt wird.

Die Reaktionszeit ist temperaturabhängig und liegt üblicherweise im Bereich von 5 bis 30 Stunden. Nach Beendigung der Reaktion wird das Reaktionsprodukt, das ein Gemisch von Isomeren darstellt, isoliert und gereinigt. Dazu wird das Reaktionsprodukt zunächst mit Eiswasser vermischt, und das Gemisch wird dann mit einem organischen Lösungsmittel, das mit Wasser nicht mischbar ist, extrahiert. Solche Lösungsmittel sind beispielsweise aromatische Kohlenwasserstoffe wie Benzol, Toluol und Xylol sowie aliphatische Chlorkohlenwasserstoffe wie Methylenchlorid und 1,2-Dichlorethan.

Der organische Extrakt wird anschließend zunächst mit Wasser und dann mit einer wäßrigen Lösung eines basischen Salzes, z.B. Natriumhydrogencarbonat und Kaliumhydrogencarbonat, oder eines Alkalihydroxids, z.B. Natriumhydroxid und Kaliumhydroxid, gewaschen und danach bei vermindertem

Druck, vorzugsweise bei einem Druck von 10 bis 30 mbar, eingedampft. Gegebenenfalls wird der Rückstand durch Destillation oder Kristallisation gereinigt. Der Anteil an 6-Acyl-2-alkylnaphthalin im resultierenden Ketongemisch wird durch Gaschromatographie bestimmt; er beträgt in der Regel mehr als 60 %. Das erfindungsgemäß erhaltene Produkt eignet sich als Ausgangsmaterial zu Herstellung von Naphthalinsäurederivaten, die ihrerseits zur Synthese von Arzneimitteln und Kunststoffen verwendbar sind.
Die Erfindung wird durch die nachstehenden Beispiele näher erläutert. Prozentangaben beziehen sich jeweils auf das Gewicht.

Beispiel 1

235 g (3,0 mol) Acetylchlorid wurden im Laufe von 30 Minuten bei einer Temperatur von 0°C in 750 g (37,5 mol) wasserfreien Fluorwasserstoff eingetropft. Zu dem erhaltenen Gemisch wurden 213 g (1,5 mol) 2-Methylnaphthalin zugefügt, und das Reaktionsgemisch wurde 20 Stunden lang bei einer Temperatur von 5°C gerührt. Danach wurde das Reaktionsgemisch in 2 l Eiswasser gegossen, und das Eiswasser-Gemisch wurde mit 1 l Toluol extrahiert. Die Toluolphase wurde mit Wasser und wäßriger Natriumhydrogencarbonatlösung neutral gewaschen und bei vermindertem Druck (20 mbar) eingedampft. Es wurden 250 g (98 Prozent der Theorie) eines Ketongemisches erhalten, das zu 78 Prozent aus 6-Acetyl-2-methylnaphthalin bestand.

Beispiel 2

306 g (3,0 mol) Acetanhydrid wurden im Laufe von 30 Minuten bei einer Temperatur von 0°C in 750 g (37,5 mol) wasserfreien Fluorwasserstoff eingetropft. Zu dem erhaltenen Gemisch wurden 213 g (1,5 mol) 2-Methylnaphthalin zugefügt, und das Reaktionsgemisch wurde 20 Stunden lang bei einer Temperatur von 18°C gerührt. Danach wurde das Reaktionsgemisch in 2 l Eiswasser gegossen, und das Eiswasser-Gemisch wurde mit 1 l Toluol extrahiert. Die Toluolphase wurde mit Wasser und mit wäßriger Natriumhydrogencarbonatlösung neutral gewaschen

und bei vermindertem Druck (20 mbar) eingedampft. Es wurden 160 g (63 Prozent der Theorie) eines Ketongemisches erhalten, das zu 74 Prozent aus 6-Acetyl-2-methylnaphthalin bestand.

Beispiel 3

36 g (0,6 mol) Essigsäure wurden in einem Autoklav im Laufe von 60 Minuten bei einer Temperatur von 20°C in 250 g (12,5 mol) wasserfreien Fluorwasserstoff eingetropft. Zu dem erhaltenen Gemisch wurden 71 g (0,5 mol) 2-Methylnaphthalin zugefügt, und das Reaktionsgemisch wurde 8 Stunden lang bei einer Temperatur von 70°C gerührt. Danach wurde das Reaktionsgemisch in 1 l Eiswasser gegossen, und das Eiswasser-Gemisch wurde mit 500 ml Toluol extrahiert. Die Toluolphase wurde mit Wasser und mit wäßriger Natriumhydrogencarbonatlösung neutral gewaschen und bei vermindertem Druck (20 mbar) eingedampft. Es wurden 82 g (89 Prozent der Theorie) eines Ketongemisches erhalten, das zu 66 Prozent aus 6-Acetyl-2-methylnaphthalin bestand.

Beispiel 4

320 g (3,0 mol) Isobuttersäurechlorid wurden im Lauf von 30 Minuten bei einer Temperatur von 0°C in 750 g (37,5 mol) wasserfreien Fluorwasserstoff eingetropft. Zu dem erhaltenen Gemisch wurden 213 g (1,5 mol) 2-Methylnaphthalin zugefügt, und das Reaktionsgemisch wurde 20 Stunden lang bei einer Temperatur von 18°C gerührt. Danach wurde das Reaktionsgemisch in 2 l Eiswasser gegossen, und das Eiswasser-Gemisch wurde mit 1 l Toluol extrahiert. Die Toluolphase wurde mit Wasser und mit wäßriger Natriumhydrogencarbonatlösung neutral gewaschen und bei vermindertem Druck (20 mbar) eingedampft. Es wurden 237 g (80 Prozent der Theorie) eines Ketongemisches erhalten, das zu 62 Prozent aus 6-Isobutyryl-2-methylnaphthalin bestand.

Patentansprüche:

1. Verfahren zur Herstellung eines 6-Acyl-2-alkylnaphthalins durch Acylierung eines 2-Alkylnaphthalins in Gegenwart eines Lösungsmittels, dadurch gekennzeichnet, daß ein 2-Alkylnaphthalin der Formel (I)

(I) 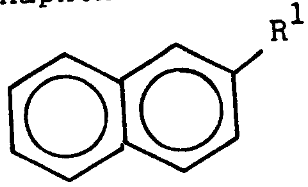 ,

in der $R^1$ einen niederen Alkylrest bedeutet, in Gegenwart von wasserfreiem Fluorwasserstoff mit einem Säurederivat der Formel (II),

(II) $R^2$-CO-X,

in der $R^2$ einen niederen Alkylrest und X eine Abgangsgruppe bedeutet, umgesetzt wird, wobei das Molverhältnis von Säurederivat zu Fluorwasserstoff 1 : 5 bis 1 : 30 und das Molverhältnis von 2-Alkylnaphthalin zu Säurederivat 1 : 1 bis 1 : 2,5 beträgt.

2. Verfahren nach Anspruch 1, dadurch gekennzeichnet, daß zunächst ein Gemisch aus dem Säurederivat und dem 2-Alkylnaphthalin bei einer Temperatur von 0 bis 5°C mit dem Fluorwasserstoff vermischt wird und anschließend die Umsetzung bei einer Temperatur von 0 bis 80°C durchgeführt wird.

3. Verfahren nach Anspruch 1, dadurch gekennzeichnet, daß zunächst das Säurederivat bei einer Temperatur von 0 bis 5°C mit dem Fluorwasserstoff vermischt wird und anschließend das 2-Alkylnaphthalin zugegeben und die Umsetzung bei einer Temperatur von 0 bis 80°C durchgeführt wird.

4. 6-Acyl-2-alkylnaphthalin, hergestellt nach dem Verfahren gemäß Anspruch 1.

Europäisches Patentamt

# EUROPÄISCHER RECHERCHENBERICHT

0212480
Nummer der Anmeldung

EP 86110951.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| P,X | US - A - 4 593 125 (KENNETH G. DAVENPORT et al.)<br>* Patentansprüche; Beispiel 3 * | 1-4 | C 07 C 49/788<br>C 07 C 45/45 |
| X | GB - A - 2 103 604 (RAYCHEM CORPORATION)<br>* Patentansprüche 1,2,5,6; Beispiel 1 * | 1,2 | |
| X | DE - A1 - 2 806 563 (HOECHST AG)<br>* Patentanspruch 1; Beispiel 8 * | 1-4 | |
| X | DE - A1 - 2 616 986 (BAYER AG)<br>* Patentanspruch 1; Seite 7, Zeilen 7-9 * | 1 | **RECHERCHIERTE SACHGEBIETE (Int. Cl. 4)** |
| A | US - A - 4 082 807 (KURT EIGLMEIER)<br>* Patentanspruch 1 * | 1 | C 07 C 49/00<br>C 07 C 45/00 |

Der vorliegende Recherchenbericht wurde für alle Patentansprüche erstellt.

| Recherchenort | Abschlußdatum der Recherche | Prüfer |
|---|---|---|
| WIEN | 10-10-1986 | REIF |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82